# EUROPEAN PATENT APPLICATION

(11) **EP 1 820 458 A1**
(43) Date of publication of application: **22.08.2007**
(21) Application number: 05811394.5
(22) Date of filing: 29.11.2005
(51) Int. Cl.: A61B 17/22, A61B 17/32, A61M 25/00

(54) **SEPARATION DEVICE FOR OBSTRUCTION IN BLOOD VESSEL**

(30) Priority: 29.11.2004 JP 2004344729
(71) Applicant: JMS Co., Ltd., Hiroshima-shi, Hiroshima 730-8652 (JP)
(72) Inventor: SHIMIZU, Yasuhiro, Hiroshima 732-0064 (JP); HAYASHI, Shuro, c/o JMS CO., LTD., Chuo-ku, Tokyo 104-0032 (JP)
(74) Representative: Thoma, Michael
(86) International application number: PCT/JP2005/021857
(87) International publication number: WO 2006/057402

(57) **Abstract**

A removing tool 1 having a tubular member 2 and guide wire 3 inserted into a vessel. The tubular member 2 has a removing portion 5 for removing an intravascular obstruction by contacting to the intravascular obstruction. The outer circumferential plane of the removing portion 5 has a spirally projecting portion 10. The tubular member 5 has an operating portion 6 held by an operator for operating the removing portion 5. A guide wire 3 guides the removing portion 5 to the position where the intravascular obstruction is formed. The operating portion 6 is operated by the operator to move the removing portion 5 back and forth in the longitudinal direction of the vessel.

## Description

### BACKGROUND OF THE INVENTION

### Technical Field

The present invention relates to a removing tool used for removing intravascular obstruction, which is formed disturbing a blood flow in a blood vessel of a living body vessel, from an inner vascular wall.

### Related Art

As a rule, an intravascular obstruction is occasionally formed and disturbs a blood flow in a human body vessel. When a thrombus, for example, is formed in a vessel as such an intravascular obstruction, a quantity of blood flowing to a peripheral portion becomes deficient to cause an ischemic disorder. As disclosed in patent document 1, a known treatment method for treating this ischemic disorder is to normalize the blood flow by softening and removing the thrombus through injecting thrombolytics into the vessel.

In addition, for example, when a venous valve, such as of a lower limb, for preventing the backflow of venous blood becomes failure, this venous blood backflow-preventing valve becomes an intravascular obstruction to make the blood flow worse, resulting in an occurrence of a varix. A known method, which is disclosed in patent document 2, for treating this varix is what is called a stripping method for removing the vein in which the varix occurred. According to this stripping method, a removing tool is inserted into the vein as a removing target by partial incision in the skin to cramp a part of the vein and, then, draw the partial vein quickly, thus removing the vein from surrounding tissues.
Patent document **1**: Japanese Patent Application Publication No. 1993-220152 (page **4**, Fig. **1**)
Patent document **2:** Japanese Patent Application Publication No. 2002-291755 (pages **5** and **6**, Figs. **4** and **5**)

### DISCLOSURE OF THE INVENTION

### Problems to be Solved by The Invention

The treatment method, as disclosed in patent document 1, that utilizes thrombolytics causes a high cost of the treatment due to a high price of thrombolytics. In addition, removal of the thrombus off the inner vessel wall using thrombolytics is occasionally imperfect depending on the status of thrombus, thereby leaving part of the thrombus on the inner vessel wall. When this status occurs, it is possible that the thrombus is again formed by the part of the thrombus left on the inner vessel wall. On the other hand, such thrombolytics cannot dissolve any intravascular obstructions other than the thrombus and, therefore, cannot be used for the treatment of, for example, the above-described varix, which is caused by a failed backflow-preventing valve of the vein.

According to the method for the treatment disclosed in patent document 2, removing the vein with the varix formed therein from the surrounding tissues causes damage to the tissues during removal, resulting in stresses such as hemorrhage and pains. Moreover, the method for the treatment disclosed in patent document 2 can only be used for the case where the varix occurs in a removable vessel such as a lower limb vein.

The present invention has been made in view of the above-described problems, and it is an object of the present invention to remove a variety of intravascular obstructions formed in the vessel from the inner vessel wall without using an expensive drug while securing the vessel, and to thereby reliably treat disorders caused by intravascular obstructions without increase in cost for treatment and without stresses.

### Means for Solving the Object

In order to achieve the above object, in the present invention, the removing tool for intravascular obstructions comprises a tubular member which has a removing portion for removing an intravascular obstruction from the inner vessel wall by contacting to the intravascular obstructions, and a guide member which guides the removing portion to a position where the intravascular obstruction is formed in the vessel.

Specifically, a first invention is drawn to the removing tool for removing an intravascular obstruction formed in the vessel in the living body from the inner vessel wall.

The removing tool comprises the tubular member, which has the removing portion, which is inserted into the vessel and then contacts to an intravascular obstruction to remove the intravascular obstruction from the inner vessel wall, and an operating portion held by an operator for operating the removing portion outside a living human body in the condition where the unit is connected to the removing portion and the removing portion has been inserted into the vessel, and the guide member, which is inserted into the tubular member to guide the removing portion to the position where the intravascular obstruction is formed in the vessel.

According to this configuration, when a variety of intravascular obstructions such as the thrombus and the venous blood backflow-preventing valve are formed in the vessel, it becomes possible to guide the removing portion of the tubular member to the position in the vessel where the intravascular obstructions have been formed, by using the guide member. Such a condition where the removing portion has been inserted into the position in the vessel where an intravascular obstruction has been formed enables easy operation of the removing portion by holding the operating portion of the tubular member by the operator in the exterior of the living body. The operation of the removing portion enables complete removal of intravascular obstructions from the internal vessel wall by contacting the removing portion to intravascular obstructions.

According to a second invention, in the first invention, the operating portion is connected to suction means for applying a negative pressure to an interior of the operating portion and a place near the removing portion of the operating portion has a thorough hole communicating with the interior of the operating portion.

According to this configuration, when the negative pressure is applied to the interior of the operating portion by actuating the suction means, the intravascular obstruction removed off the internal vessel wall by the removing portion is sucked into the inside through the thorough hole of the operating portion.

According to a third invention, in the first or the second invention, the tubular member has an extending portion extending to the exterior of the living body in the condition where the removing portion is located in the position where the intravascular obstruction is formed in the vessel.

According to this configuration, when the removing portion of the tubular member is inserted to the position where the intravascular obstruction is formed in the vessel, the extending portion of the tubular member extends to the exterior of the living body. This enables the operator to hold both the extending portion and the operating portion by hand and to use the extending portion and the operating portion to operate the removing portion from both sides of the vessel in the longitudinal direction, making it possible to stabilize the removing portion in the vein during the operation.

According to a fourth invention, in the third invention, an end of the extending portion in the direction of insertion has a tapering plane tapering toward the end side.

According to this configuration, when the tubular member is inserted into the vessel, the tapered plane of the tubular member contacts to the internal vessel wall, thereby making the insertion smooth.

According to a fifth invention, in any one of the first to the fourth inventions, an outer circumferential plane of the removing portion has a projecting portion projecting from the outer circumferential plane to extend spirally.

According to this configuration, since the projecting portion extends spirally, one movement of the removing portion, which is inserted into the position where the intravascular obstruction is formed in the vessel, to the longitudinal direction of the vein brings the tips of the projecting portion into frequent contact to the intravascular obstruction. Hence, the intravascular obstruction can be removed efficiently from the inner vessel wall. In addition, since the projecting portion is spirally shaped, a spiral groove is formed on the outer circumference of the removing portion, resulting in that the intravascular obstruction, when removed from the inner vessel wall, is contained in the groove instead of being attached to the tips of the projecting portion. As a result, the tips of the projecting portion can be always exposed, enabling make more efficient removal of obstructions.

### Effects of the Invention

According to the first invention, the removing portion, which removes an intravascular obstruction from the inner vessel wall, can be guided by the guide member to the position where the intravascular obstruction is formed in the vessel. Therefore, a variety of intravascular obstructions can be removed from the inner vessel wall by using the removing portion. In this step, when intravascular obstructions are removed from the inner vessel wall, no expensive drug is required while securing the vessel. Therefore, disorders caused by intravascular obstructions can be reliably treated at a low cost and with a low stress.

According to the second invention, the operating portion is internally applied with a negative pressure and the place near the removing portion of the operating portion has a thorough hole communicating with the interior of the operating portion. Therefore, intravascular obstructions removed from the inner vessel wall are sucked into the interior of the removing portion from the thorough hole to be reliably removed from the vessel.

According to the third invention, the tubular member has an extending portion extending to the exterior of the living body. Therefore, the removing portion can be stably operated from both the longitudinal sides of the vessel to enable it to remove more reliably intravascular obstructions from the inner vessel wall.

According to the fourth invention, the end of the extending portion in the direction of insertion has a tapering plane tapering toward the end side. Therefore, the tubular member can be smoothly inserted into the vessel to enable to remove intravascular obstructions with a low stress.

According to the fifth invention, the removing portion has a projecting portion extending spirally to the outer circumferential plane of the removing portion. This enables reliable removal of intravascular obstructions from the inner vessel wall, without the need for increasing the frequency of operation and movement of the removing portion in the vessel. Moreover, it is possible to expose always the tips of the projecting portion, enabling efficient removal of intravascular obstructions.

### BRIEF DESCRIPTION OF THE DRAWINGS

Fig. **1** is a side view of a removing tool for intravascular obstructions, according to an embodiment of the present invention.
Fig. **2** is a side view of the removing tool for intravascular obstructions, showing the status where a suction apparatus is connected to the removing tool.
Fig. **3** is a partial sectional view of the removing tool for intravascular obstructions, showing an enlarged removing portion of a tubular member.
Fig. **4** is a diagram illustrating the case where a venous blood backflow-preventing valve is removed from an inner vessel wall: Fig. **4**(a) shows the condition of a guide wire inserted into a vein; and Fig. **4**(b) shows the condition of the tubular member inserted into the vein.
Fig. **5** is a diagram corresponding to Fig. **1**, according to a modified example **1** of the embodiment.
Fig. **6** is a diagram corresponding to Fig. **4**(b), according to a modified example **2** of the embodiment.
Fig. **7** is a diagram corresponding to Fig. **1**, according to a modified example **3** of the embodiment.
Fig. **8** is a diagram corresponding to Fig. **1**, according to a modified example **4** of the embodiment.
Fig. **9** is a diagram corresponding to Fig. **1**, according to a modified example **5** of the embodiment.

### Description of the reference numerals.

- 1: Removing tool
- 2: Tubular member
- 3: Guide wire (guide member)
- 5: Removing portion
- 6: Operating portion
- 7: Extending portion
- 10: Projecting portion
- 15: Suction apparatus (suction means)
- 17: Thorough hole
- 22: Tapered plane
- A: Vein
- B: Venous blood backflow-preventing valve (intravascular obstructions)

### DESCRIPTION OF THE PREFERRED EMBODIMENTS

Preferred embodiments of the present invention will be described in detail as follows in conjunction with the drawings.

### Embodiment 1

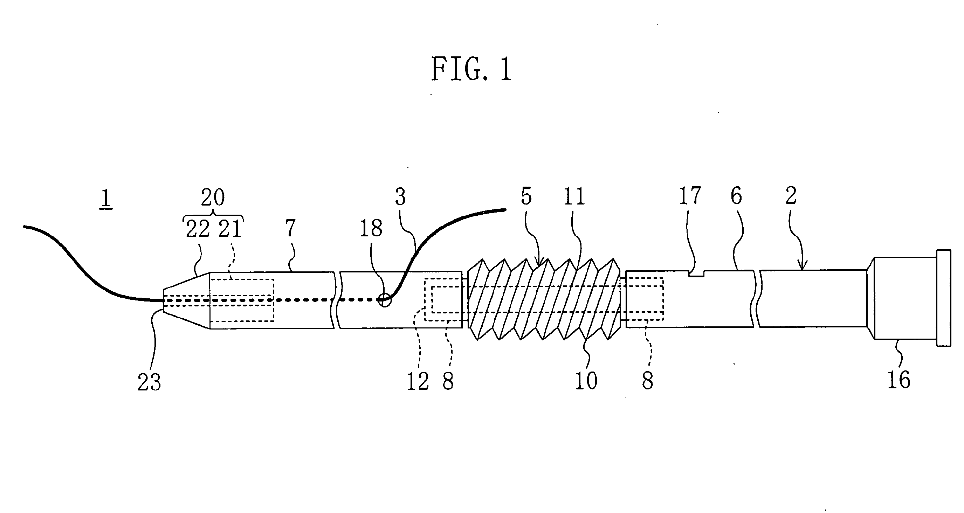

Fig. 1 shows a removing portion 1 for intravascular obstructions, according to embodiment 1 of the present invention. The removing portion 1 has a tubular member 2 to be inserted into the vessel and a guide wire 3 as a guide member for guiding the tubular member 2 in the vessel.

The tubular member 2, as a whole, is formed to extend in the direction of insertion in the vessel, the total length being set at from approximately 700 mm to 800 mm. In addition, the tubular member 2 has a cylindrical removing portion 5 for removing intravascular obstructions formed in the vessel from the inner vessel wall, a long operating portion 6 extending from one end in an axial direction of the removing portion 5, and a long extending portion 7 extending from the other end in the axial direction of the removing portion 5.

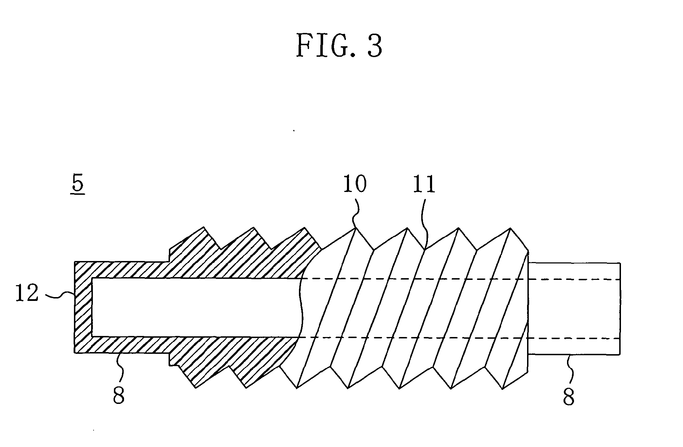

The removing portion 5 is made by molding a resin material such as an elastomer resin. The length of the removing portion 5 is set at from approximately 20 mm to 30 mm in the axial direction. As also shown in Fig. 3, the removing portion 5 is set so that the inner diameter is the same throughout the axial direction, from one end to the other end, while the outer diameter has smaller ends in the axial direction. The removing portion 5 has at each end in the axial direction a small diameter portion 8 that is thinner than a middle portion. The middle portion of the removing portion 5 in the axial direction has a projecting portion 10 integrally formed, projecting from an outer circumference of the removing portion 5, and extending spirally along the outer circumference. The projecting portion 10 has a triangle section having an apex on the projecting tip. Thus, the outer circumference of the removing portion 5 has a spirally extending groove 1. Further, the extending portion 7 side of the removing portion 5 is blocked by a blocking portion 12.

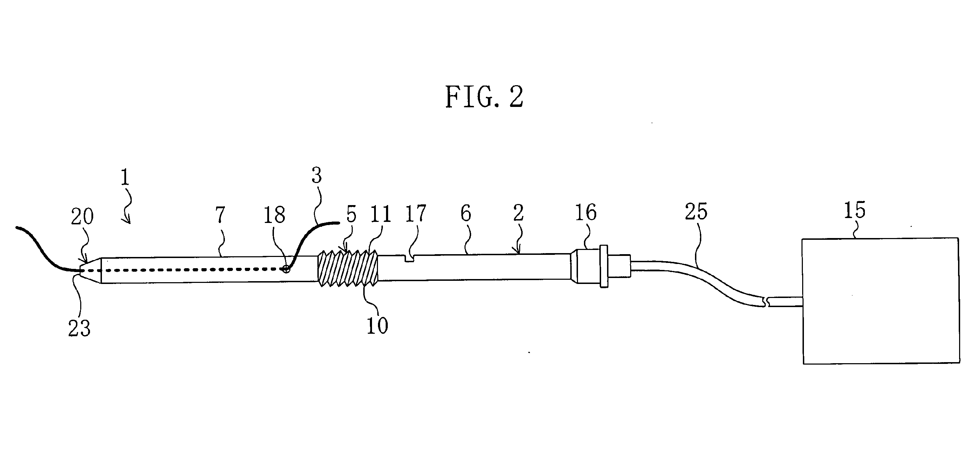

For the operating portion 6, a resin material softer than the resin material constituting the removing portion 5 is formed into a tube-like shape to allow for easy bending during insertion into the vessel. The inner diameter of the end of the removing portion 5 side of the operating portion 6 is set slightly smaller than the outer diameter of the small diameter portion 8 of the removing portion 5. The small diameter portion 8 of the operating portion 6 side of the removing portion 5 is held in the condition of insertion into and fitting to the interior of the end of the operating portion 6. This makes the removing portion 5 integral with the operating portion 6. The other end of the operating portion 6 opposite to the removing portion 5 has a connecting portion 16 to be connected to a suction apparatus 15 (shown in Fig. 2) mentioned later. The connecting portion 16 is formed in a tubular shape having a diameter larger than that of the middle portion of the operating portion 6 in the longitudinal direction. In addition, a surrounding wall of the operating portion 6 has a thorough hole 17 in the vicinity of the end of the operating portion 6 at the removing portion 5 side in order to provide a communication between the interior and the exterior of the operating portion 6.

For the extending portion 7, the same resin material as that of the operating portion 6 is formed into a tubular shape. The inner diameter of the end of the extending portion 7 at the removing portion 5 side is slightly smaller than the outer diameter of the small diameter portion 8 of the removing portion 5, while the small diameter portion 8 of removing portion 5 at the extending portion 7 side is held in the condition of insertion into and fitting to the interior of the end of the extending portion 7. This makes the removing portion 5 integral with the extending portion 7. In addition, the surrounding wall of the extending portion 7 has an inlet hole 18 in the vicinity of the end of the extending portion 7 at the removing portion 5 side in order to insert the guide wire 3 therethrough.

On the other hand, the end of the extending portion 7 opposite to the removing portion 5 has an end cap portion 20. The end cap portion 20 also constitutes the tubular member 2. For the end cap portion 20, a resin material harder than the extending portion 7 is formed into a roughly cylindrical shape. The inner diameter of the end cap portion 20 is slightly smaller than the inner diameter of the removing portion 5 and the inner diameter of the extending portion 7. The end cap portion 20 has an insertion portion 21 to be inserted into the side of the extending portion 7 opposite to the removing portion 5. The outer diameter of the insertion portion 21 is slightly larger size than the inner diameter of the extending portion 7. The insertion portion 21 is held in the condition of insertion into the extending portion 7. This makes the extending portion 7 integral with the end cap portion 20. The side of the end cap portion 20 opposite to the insertion portion 21 has a tapered plane 22 tapering toward a distal position of the insertion portion 21. The tapered plane 22 is formed to be continuous with respect to the end of the extending portion 7 in the condition in which the insertion portion 21 is inserted into the extending portion 7. The end of the tapered plane 22 is connected to a flat plane 23 extending in the direction approximately orthogonal to the axial direction of the end cap portion 20. The flat plane 23 has an opening through which the guide wire 3 is inserted.

The suction apparatus 15 connected to the connecting portion 16 constitutes the suction means of the present invention and is connected to the connecting portion 16 through a connecting pipe 25. The suction apparatus 15 has a known structure commonly used in the medical field.

On the other hand, the guide wire 3 is made by twining a multiplicity of fine steel wires and has a flexibility enabling easy bending during insertion into the vessel. The diameter of the guide wire 3 is smaller than the inner diameter of each portion of the tubular member 2, and the length is longer than the length of the tubular member 2.

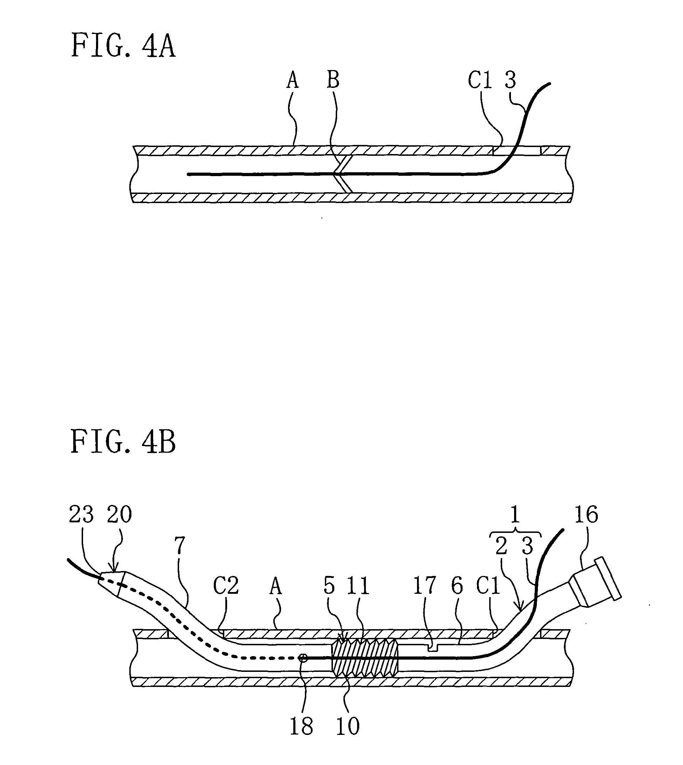

Next, the treatment of a varix formed in a vein A of a lower limb by using the above-constituted removing tool 1 for intravascular obstructions will be described with reference to Fig. 4. The varix results from a deteriorated flow of blood caused by a failed venous blood backflow-preventing valve B in vein A, turning the venous blood backflow-preventing valve B into an intravascular obstruction. On the other hand, in removing tool 1 used for this case, the outer diameters of the operating portion 6 and the extending portion 7 are slightly smaller than the inner diameter of the vein A and the distance between the tips of the projecting portion 10 in the radial direction of the removing portion 5 is slightly larger than the inner diameter of the vein A.

First, partial skin incision is carried out to expose the vein A having the varix at a further central side (an upstream side in the blood flow direction) than the varix. Next, as shown in Fig. 4(a), the exposed part of the vein A is incised to make a central side-incised portion C1. Then, the guide wire 3 is inserted into the vein A at the peripheral side (a downstream side in the blood flow direction) through the central side-incised portion C1. At this time, the operator operates the guide wire 3a at the proximal side thereby facilitating selective insertion of the end of the guide wire 3 into a desired vein branch among a multiplicity of vein branches.

When the end of the guide wire 3 is sufficiently past the point of the vein A where the varix is formed, insertion of the guide wire 3 is discontinued. Then, a point of the skin corresponding to the end of the guide wire 3 is partially incised, and a point of the vein corresponding to the end of the guide wire 3 is also incised, thus forming a peripheral side-incised portion C2 (shown in Fig. 4(b)). Then, the end of the guide wire 3 is pulled out of the skin through the peripheral side-incised portion C2 of the vein A and the dissected position of the skin.

Next, the tubular member 2 is inserted into the vein A through the central side-incised portion C1 of the vein A. At this time, first, the end of guide wire 3, which is projected from the central side-incised portion C1, is inserted into the extending portion 7 through the end cap portion 20 of the tubular member 2. Then, the end cap portion 20 is inserted into the vein A through the central side-incised portion C1, and the extending portion 7, removing portion 5, and the operating portion 6 are inserted into the vein A by hand in this order. By this operation, the end cap portion 20 and the extending portion 7 are guided by the guide wire 3 to pass through the point of the vein A where the varix is formed, and finally to project to the exterior of the skin through the peripheral side-incised portion C2. The end cap portion 20 projecting through the peripheral side-dissected part C2 is pulled out of the skin and the operating portion 6 is pushed to the interior of the vein A in the direction of insertion so that the extending portion 7 is guided by the guide wire 3 further toward the direction of insertion. The removing portion 5, which is continuous to the extending portion 7, is also indirectly guided by the guide wire 3, which enables it to position the removing portion 5 in the vicinity of the failed venous blood backflow-preventing valve B.

In this state, the operating portion 6 and the extending portion 7 respectively project from the central side-incised portion C1 and the peripheral side-incised portion C2 of vein A to the exterior of the skin. The operator can handle both the operating portion 6 and the extending portion 7 by hand to allow the removing portion 5 to move back and forth in the vein A in the extension direction for the vein A. Thus, the removing portion 5 can be operated from both sides of the axial direction, and hence, the removing portion 5 can be stabilized in vein A during operation.

Operating the removing portion 5 in the above-described manner brings the tips of the projecting portion 10 into contact with the blood backflow-preventing valve B, thereby removing the blood backflow-preventing valve B off the inner wall of the vein A. In removing the blood backflow-preventing valve B off the inner wall of the vein A, since the distance between the tips of the projecting portion 10 is larger than the inner diameter of the vein A in the radial direction of the removing portion 5, the tips of the projecting portion 10 reliably come into contact with the blood backflow-preventing valve B. Further, since the projecting portion 10 is spirally shaped, only a single movement of the removing portion 5 provides frequent contact between the tips of the projecting portion 10 and the blood backflow-preventing valve B.

Moreover, when a negative pressure is applied to the interior of the operating portion 6 by actuating the suction apparatus 15 while moving the removing portion 5 in the vein A, the blood backflow-preventing valve B removed from the inner wall of vein A is sucked into the tubular member 2 through the thorough hole 17 to the suction apparatus 15 through the operating portion 6, thus removing the blood backflow-preventing valve B from the interior of the vein A. The suction apparatus 15 may be actuated following removal of the blood backflow-preventing valve B from the inner wall of vein A.

Next, although not shown, the case where the treatment of an ischemic disease caused by a thrombus formed in the vein of a lower limb by using the removing tool 1 for intravascular obstructions will be described below. The removing tool 1 used in this case is such that the size of each portion is set in the same manner as in the case of the treatment of the varix as described above.

First, partial skin incision is carried out to expose a further central side of the vein where the varix is formed than the thrombus followed by incision of the exposed part of the vein. Then, the guide wire 3 is inserted from the central side-incised portion of the vein toward the peripheral side, and when the end of the guide wire 3 is sufficiently past the thrombus, the insertion of the guide wire 3 is discontinued.

Subsequently, in the same manner as that of the treatment of the varix as described above, when the tubular member 2 is inserted into the vein, the end of the tubular member 2 is guided by the guide wire to penetrate through the thrombus and the removing portion 5 of the tubular member 2 is guided by the guide wire 3 to the position in the vein where the thrombus is formed. Then the operator handles the operating portion 6 of the tubular member 2 by hand to allow the removing portion 5 to move back and forth in the vein in the extending direction for the vein. By this step, the tips of the projecting portion 10 of the removing portion 5 contacts the thrombus to remove the thrombus from the inner wall of the vein. In removing the thrombus from the inner wall of the vein A, reliable removal becomes possible by designing the size and shape of the projecting portion 10, similarly to the case of removing the blood backflow-preventing valve B as described above. In addition, sucking the thrombus removed from the inner wall of the vein by using the suction apparatus 15 results in removal of the thrombus from the interior of the vein.

As described above, the removing tool 1 is comprised of the tubular member 2, which has the removing portion 5 for removing the blood flow-preventing valve B and the thrombus, which prevent blood flow, from the inner wall of the vein A, and the guide wire 3 for guiding the removing portion 5 in the vein A. Thus, the removing portion 5 can be reliably guided by the guide wire 3 to the position in the vein A where the blood backflow-preventing valve B or the thrombus is formed, enabling the removing portion 5 to remove the blood backflow-preventing valve B or the thrombus from the inner wall of the vein A. By this step, when diseases caused by a variety of intravascular obstructions formed in the vein A are treated, it is not required to use an expensive thrombolytics and to extract and remove the vein A. This suppresses an increase in treatment cost and the treatment can be reliably conducted in low stresses.

Also, the thorough hole 17 is provided on the circumferential wall of the operating portion 6 in the vicinity of the removing portion 5, and a negative pressure is applied to the interior of the operating portion 6 from the suction apparatus 15. Thus, the blood backflow-preventing valve B or the thrombus removed from the inner wall of vein A can be reliably removed from the interior of the vein A.

In treating the varix, the operating portion 6 and the extending portion 7 are projected from the skin to allow the operator to hold the portions so that the removing portion 5 can be handled at both ends in the axial direction. This makes the removing portion 5 stable in vein A during the operation to achieve reliable removal of the blood flow-preventing valve B from the inner wall of the vein A.

Since the end cap portion 20 is formed in a tapered shape, when the tubular member 2 is inserted into the vein A, the tubular member 2 is not caught in the inner wall of the vein A. Thus, the tubular member 2 can be smoothly inserted into the vein A to enable it to remove the blood backflow-preventing valve B and the thrombus in low stresses.

In addition, since the removing portion 5 has the spirally formed projecting portion 10, only a single movement of the removing portion 5 in vein A, for example, provides frequent contact between the tips of the projecting portion 10 and the blood backflow-preventing valve B or the thrombus, thereby providing efficient removal of the blood backflow-preventing valve B or the thrombus. In addition, since the spiral groove 11 is provided on the outer circumference of the removing portion 5, the blood backflow-preventing valve B or the thrombus removed from the inner wall of vein A is not left on the tips of the projecting portion 10 but contained in the groove 11. As a result, the tips of the projecting portion 10 can be always exposed. This alone realizes efficient removal of the blood backflow-preventing valve B or the thrombus.

Further, since the spiral groove 11 is in a spiral shape, the blood backflow-preventing valve B or the thrombus contained in the groove 11 are, through the back-and-forth movement of the removing portion 5 in the vein A, moved through the groove 11 to the operating portion 6 side and the extending portion 7 side of the groove 11 to be finally discharged out of the groove 11. This reliably prevents clogging of the projecting portion 10.

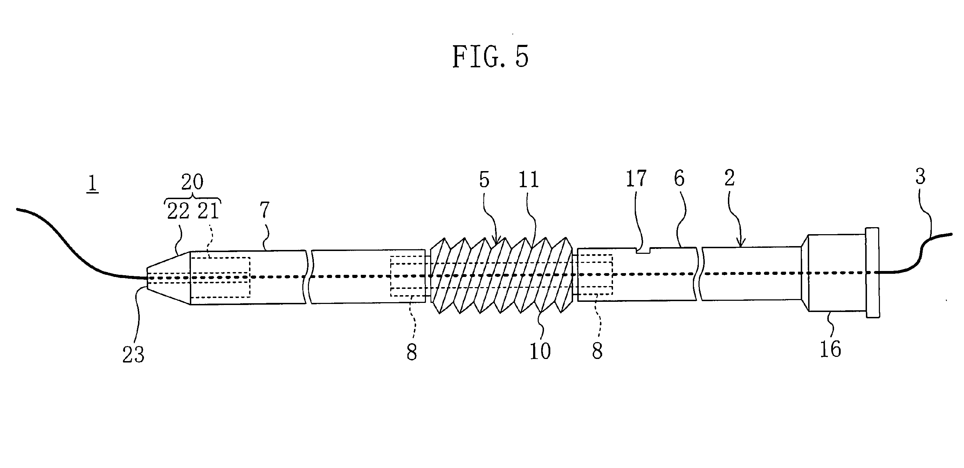

According to this embodiment, the guide wire 3 is inserted into only the cap portion 20 and the extending portion 7. However, as in a modification example 1 shown in Fig. 5, the guide wire 3 may be inserted throughout the longitudinal direction of the tubular member 2.

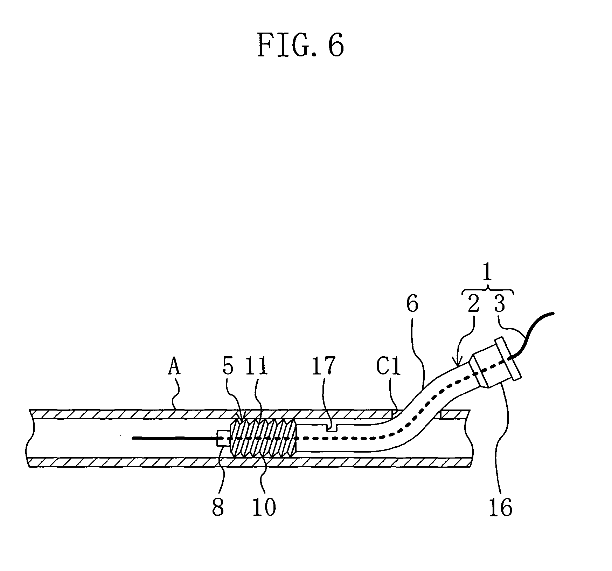

Further, according to this embodiment, the tubular member 2 has the extending portion 7. This extending portion 7 may be omitted as in a modification example 2 shown as shown in Fig. 6. In this case, the end of removing portion 5 opposite to the operating portion 6 is open so that the guide wire 3 is inserted across the removing portion 5 and the operating portion 6.

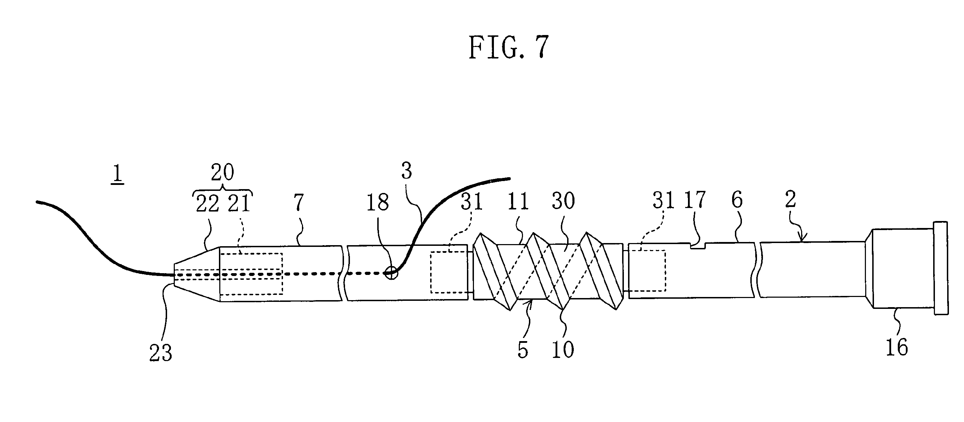

According to this embodiment, the projecting portion 10 is formed integrally with the main body of the removing portion 5. However, as in a modification example 3 shown as shown in Fig. 7, the projecting portion 10 may be separately provided using, for example, elastomer resin, followed by adhesion to a cylindrical unit 30 with, for example, an adhesive. The cylindrical unit 30 is composed of the same material as that of the operating portion 6 and the extending portion 7. Both ends of the cylindrical unit 30 in the longitudinal direction has small diameter portions 31 similar to the small diameter portion 8. Each of the small diameter portions 31 is inserted into the operating portion 6 and the extending portion 7 so that the cylindrical unit 30, the operating portion 6, and the extending portion 7 are integral with each other.

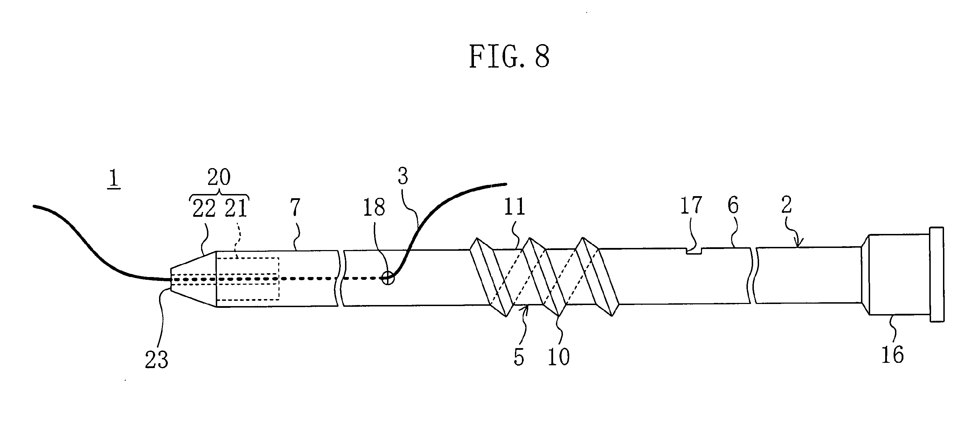

In addition, as in a modification example 4 shown in Fig. 8, the operating portion 6 of the tubular member 2, the main body of the removing portion 5, and the extending portion 7 may be integrally formed, and the projecting portion 10 may be such that as in the modification example 3, a separately provided member is adhered to the main body of the removing portion 5. Integral forming of the operating portion 6, the main body of the removing portion 5, and the extending portion 7 provides approximately uniform softness throughout the tubular member 2. As a result, during insertion into the vein A, when, for example, the tubular member 2 passes a bent portion of the vein A, there is substantially no change in an inserting force. This improves the operability of the removing tool 1.

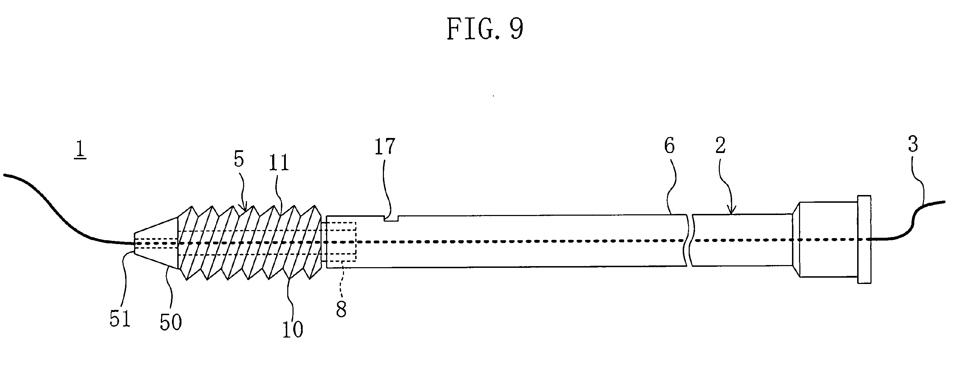

As in a modification example 5 shown in Fig. 9, the removing portion 5 may be placed at the end of the tubular member 2 in the direction of insertion. The small diameter portion 8 of the removing portion 5 is inserted into and fitted to the interior of the end of the tubular member 2. On the end side of the removing portion 5 in the direction of insertion, a tapered plane 50 is formed to taper distally. The end of the tapered plane 50 is connected to a flat plane 51 extending in the direction approximately orthogonal to the axial direction of tubular member 2. On the flat plane 51, an opening through which the guide wire 3 passes is formed. Since in the removing tool 1 according to the modification example 5 the removing portion 5 is located at the end of tubular member 2 in the direction of insertion, only a single incised portion is necessary on the skin so that the guide wire 3 is inserted into the vein A through the incised portion followed by insertion of the removing portion 5 and the tubular member 2 and then by holding of the tubular member 2 to operate the removing portion 5. Thus, since only a single incised portion is necessary, the treatment involves low stresses.

According to this embodiment, the projecting portion 10 is continuous to the removing portion 5 in a circumferential direction. The removing portion 5 may, for example, have projections not continuous in the circumferential direction. Further, the sectional shape of the projecting portion 10 is not restricted to a triangle, but may be, for example, a rectangle.

In treating the varix, following removal of the blood backflow-preventing valve B from the inner wall of vein A, the removing portion 5 may be further allowed to move back and forth to cause the projecting portion 10 to pass the inner wall of vein A with friction. By this way, following removal of the tubular member 2 and the guide wire 3 out of the vein A, when the vein A is held with pressure from the exterior of the skin, because of the losing of the blood backflow-preventing valve B and friction of the inner wall of the vein A, the vein A can be completely crashed to adhere inner walls to each other. This blocks the blood flow in vein A, thereby degenerating the vein A.

When the blood backflow-preventing valve B or the thrombus is removed from the inner wall of the vein A, physiological saline, for example, may be introduced into the vein A. Specifically, for example, a syringe, not shown, filled with physiological saline is connected to the connecting portion 16 of the operating portion 6, and after the blood backflow-preventing valve B or the thrombus is removed from the inner wall of vein A by the removing portion 5, the syringe is operated to supply the physiological saline to the vein A through the thorough hole 17. This facilitates the movement, in the vein A, of the blood backflow-preventing valve B or the thrombus removed from the inner wall of vein A. Thus, the blood backflow-preventing valve B and the thrombus can be more reliably sucked by suction apparatus 15.

According to this embodiment, the suction means is constituted by the suction apparatus 15. This suction means may be constituted by a syringe, for example.

### INDUSTRIAL APPLICABILITY

As described above hereinbefore, the removing tool for intravascular obstructions according to the present invention can be used for, for example, treating a varix caused by a failed blood backflow-preventing valve in the vein.

## Claims

1. A removing tool for removing intravascular obstructions formed in a vessel of a living human body from an inner vessel wall, the removing tool comprising:
a tubular member having: a removing portion inserted into the vessel for contacting to an intravascular obstruction to remove the intravascular obstruction from the inner vessel wall; and an operating portion held and operated by an operator for operating the removing portion outside a living human body in a condition where the operating portion is connected to the removing portion and the removing portion has been inserted into the vessel; and
a guide member inserted into the tubular member to guide the removing portion to a position where the intravascular obstruction is formed in the vessel.

2. The removing tool for removing intravascular obstructions according to claim 1, wherein:
the operating portion is connected to suction means for applying a negative pressure to an interior of the operating portion; and
a place near the removing portion of the operating portion has a thorough hole communicating with an interior of the operating portion.

3. The removing tool for removing intravascular obstructions according to claim 1 or 2, wherein the tubular member has an extending portion extending to an exterior of the living human body in the condition where the removing portion is located in a position where the intravascular obstruction is formed in the vessel.

4. The removing tool for removing intravascular obstructions according to claim 3, wherein an end of the extending portion in a direction of insertion has a tapering plane tapering toward an end side.

5. The removing tool for removing intravascular obstructions according to any one of claims 1 to 4, wherein an outer circumferential plane of the removing portion has a wire projecting from an outer circumferential plane to extend spirally.
